# EUROPEAN PATENT SPECIFICATION

(11) **EP 0 881 911 B1**
(45) Date of publication and mention of the grant of the patent: **02.05.2003**
(21) Application number: 97905540.7
(22) Date of filing: 20.02.1997
(51) Int. Cl.: A61K 39/39

(54) **IMMUNOGENIC COMPLEX, ISCOM, FOR USE IN PREPARING A VACCINE FOR JUVENILE INDIVIDUALS**
IMMUNOGENER KOMPLEX, ISCOM, ZUR HERSTELLUNG EINES IMPFSTOFFS FÜR JUGENDLICHE
COMPLEXE IMMUNOGENIQUE, COMPOSE IMMUNOSTIMULATEUR POUR LA PREPARATION D'UN VACCIN POUR DE JEUNES ANIMAUX

(30) Priority: 21.02.1996 SE 9600646
(43) Date of publication of application: 09.12.1998
(73) Proprietor: Morein, Bror, S-755 91 Uppsala (SE)
(72) Inventor: Morein, Bror, S-755 91 Uppsala (SE)
(74) Representative: Fagerlin, Heléne
(86) International application number: SE9700288
(87) International publication number: WO97030727

(56) References cited:
- US-A- 5 034 159
- US-A- 5 223 254
- VACCINE, Volume 11, No. 13, 1993, E.T.S. BEN AHMEIDA et al., "Immunopotentiation of Local and Systemic Humoral Immune Responses by ISCOMs, Liposomes and FCA: Role in Protection Against Influenza A in Mice", page 1302.
- VACCINE, Volume 11, No. 2, 1993, ALAN J. HUSBAND, "Novel Vaccination Strategies for the Control of Mucosal Infection", page 107.
- MED. MICROBIOL. IMMUNOL., Vol. 183, 1994, K. SCHEEPERS et al., "Protection of Mice Against an Influenza Virus Infection by Oral Vaccination With Viral Nucleoprotein Incorporated Into Immunostimulating Complexes", pages 265-278.
- VIRUS RESEARCH, Volume 32, 1994, B.R.MURPHY et al., "An Update on Approaches to the Development of Respiratory Syncytial Virus (RSV) and Parainfluenza Virus Type 3 (PIV3) Vaccines", pages 13-36.
- SCHNEEWEISS & ROBBLER: 'Reifungsprobleme des Immunsystems im Kindesalter' ALLERG. IMMUNOL. (LEIPZ.) vol. 22, no. 3, 1976, pages 257 - 263

## Description

This invention involves immunogenic complexes in the form of iscom or iscom matrix for use in preparing vaccines that stimulate immune responses in juveniles. A problem concerning juveniles is that they are not immunocompetent, that is, they seem to lack the ability to respond immunologically when they are exposed to antigenic stimuli. Moreover, juvenile individuals have often already obtained antibodies and possibly also cellular elements belonging to a passively transferred immune defense from the mother to the off-spring, so-called maternal immunity.

It is well known that the vaccines in use today for young individuals with maternal immunity do not evoke immune response, at least not an immune response that gives sufficient protection. Maternal immunity is transmitted from the mother to the child through navel blood or through breast milk, in some animals i.e. cattle and horse especially through cholostrum during the first 1-2 days after birth. In general, maternal immunity in the newborn is measured as serum antibodies directed toward the infectious agent in question. In humans and animals alike, the neonatal period constitutes a serious problem in connection with vaccination against various infectious agents from which young individuals need extra protection because they fail to respond immunologically due to an immature immune system. In this context it should be pointed out that the purpose of maternal immunity is to protect the mother's young. Maternal immunity gives protection from birth and a short time afterward, but from a certain point the protection subsides and the infant is unprotected and susceptible to infection. During this period immunocompetence is also being developed, which in mice has reached an adult level at three weeks of age, but which in larger animals and human beings takes a longer time. It is quite clear that there exists a combination of a poorer ability for young individuals to respond immunologically that increases with age and a blocking effect of the maternal immunity that subsides with age. During the break-off period, the vaccinations available today are therefore without effect. For example, vaccination against the measles virus should not be given before one year of age, since the vaccine is without effect before then. In Africa as in other developing areas, children are often infected with the measles virus before one year of age, which gives rise to serious illness, often resulting in complications that can lead to death. In the third world, infections with the measles virus during the first year after birth is one of the most common causes of infant mortality.

In veterinary medicine, it is known that puppies do not generally respond to vaccination before 12 weeks of age. Vaccination against distemper is therefore recommended to be administered at this age. In addition to distemper, there is a need to vaccinate juvenile immunologically immature dogs with maternal immunity against parvovirus. The same problem exists when cats are to be vaccinated against panleukopenia caused by a parvovirus closely related to the dog parvovirus. Kennel owners and cat breeders can experience serious problems involving infections with such viruses. The infection lingers on by infecting the young during the immunologically immature period when they still have maternal antibodies that prevent the vaccination from giving the desired effect. In the same way, attempts have been made to vaccinate horses against the herpes equi 2 virus (Heq2) using conventional types of vaccines during the period when the foal has maternal immunity. However, the conventional vaccine was ineffective and no antibody response could be detected in the serum against this virus, and to an even lesser degree could any protection against the infection or illness be detected.

De Vries et al. suggest that iscoms could possibly be a suitable vaccine strategy in individuals with maternal immunity based on results from experiments where adults, monkeys and rats have been injected with antibodies against a pathogen (Measles virus and a subsequent challange infection). No experimental data are, however, presented about juveniles where the problem is real. No data were given to tell about the problems of the juveniles indicating that the author did not even know about the immune system. It has unexpectedly been demonstrated that iscom or iscom matrix may be used in preparing a vaccine that elicits immune response in juvenile individuals with immature immune system with or without maternal immunity. This is examplified in two days old mice one week old seals, two weeks old foals and one week old lambs.

Juvenils are, according to this invention, young mammals that are immunoincompetent from the point of view that they do not respond to vaccination, i.e. they have a reduced capacity to respond immunologically when they are exposed to antigenic stimuli. Moreover they may or may not have maternal immunity, i.e. antibodies from their mothers.

Iscom contains at least one glycoside, at least one lipid and at least one type of antigen substance, particularly proteins and peptides. These complexes enhance the immunogenicity of the included antigens and may also contain one or more immunomodulatory (adjuvant-active) substances, as described in EP 0 109 942 B1 EP 0 242 380 B1 and EP 0 180 564 B1.

Matrix contains at least one glycoside, an adjuvant-active substance and at least one lipid. Matrix has an immunostimulating effect when administered at the same time as the antigen substances, see EP 0 436 620 B1.

In accordance with the invention, the complexes may be composed of iscom complexes with antigens that are integrated by hydrophobic interactions in the iscom complex or coupled to a ready-made iscom complex.

They can also be composed of iscom matrix to which an antigen is coupled and then by definition is converted to iscom. The complex may also be iscom matrix which is mixed with the antigen or kept in separate entities and administered at the same time.

It is also possible to use iscom complexes that contain an antigen or molecule that functions as a mucus targeting molecule through the mucous membranes, along with another antigen that cannot penetrate the mucous membranes and cannot target lymphatic tissue in the local mucosa, as described in the Swedish patent application 9600647-3. Furthermore, iscom complexes may be used that contain receptor substances for antigen or targeting molecules as described in the Swedish patent application 9600648-1.

Antigens come from microorganisms such as bacteria, viruses or parasites, particularly those given in EP 0 109 942 B1. In particular, they are antigen substances such as proteins and peptides or carbohydrates, carbohydrate structures, such as glycolipides, glycopeptides or -proteins.

One such example is bacterial toxins like the cholera toxin or its subunit B (CTB), the heat-labile toxin in E. coli, or its subunit B (LTB). Other examples are enveloped proteins from viruses or proteins from bacteria that are mucous-targeting and which infect the respiratory passages, such as the influensa virus, respiratory syncytial virus (RSV), the coronavirus, the Astrovirus, Norwalk virus ,the herpes virus, pox virus, membrane proteins from Mycoplasma and fimbriae from various bacteria such as Haemophilus or surface proteins from non-enveloped viruses and bacteria that infect the intestines, such as adenovirus, reovirus, parvovirus, rotavirus and fimbriae from Escherichia coli (K88, K99, K98I-B), Shigella, Clamydia and mycoplasma.

Such microorganisms may be the measles, German measles and chicken pox viruses; Mycoplasma pneumoniae, myccplasma mycoides, Neisseria meningitidis, Neisseria gonorrhoeae, Vibrio cholerae, Chlamydia psittaci, Chlamydia pneumonia, Salmonella typhi, Streptococcus mutans, Helicobacter pylori, Streptococcus pyogenes, Corynebacterium diphtheriae, Mycobecterium tuberculosis, Yersinia pestis, Salmonella typhi, Borrelia burgdorferi, Plasmodium vivax, Plasmodium falciparium, Toxoplasma gondii, Trypanosoma brucei, Gardia lambiia and Entamoeba histolytica; and Cryptococcus neoformans and Histoplasma capsulatum.

Further examples are gB and gD in various Herpes viruses such as Herpes Simplex 1 and 2, bovine herpes virus 1, picorna virus, gp 120 and gp 160 in HIV-1 or the corresponding protein in HIV-2, as well as antigens from other retroviruses, hepadna viruses pox virus, adenovirus, cardivirus, togavirus, flavivirus, iridiovirus, birnavirus, parvovirus, popovavirus, picornavirus, calicivirus, astrovirus, arenavirus, bunyavirus, ortomyxovirus, paramyxovirus, G-protein from the rabies virus, or from bacteria such as salmonella, E. coli, Shigella, clamydia or mycoplasma, as well as various recombinant products or synthetic antigens. In particular, distemper, foot-and-mouth disease virus, parvoviruses, hog cholera virus and Herpes equi 1, 2 and 4 viruses are mentioned.

In those cases where the complexes are iscoms, they can be prepared as described in the European patent EP 0 109 942 B1. Thus, viruses, mycoplasma, bacteria, parasites, animals cells containing antigens or antigen determinants, particularly proteins or peptides or isolated examples, that have hydrophobic or amphiphatic regions, are mixed with one or more solubilising agents, whereby complexes are formed between antigens or antigen determinants and solubilising agents, after which the antigens or determinants are separated from the solubilising agent, or is separated from the solubilising agent and is directly transferred to a glycoside solution, containing cholesterol, phospholipids and one or more glycosides with hydrophobic or hydrophilic domains in a concentration of a minimum of the critical micellar concentration, thereby forming a protein complex which is isolated and purified.

The starting material may come from whole microorganisms containing antigens to which, more or less purified mucus targeting molecules, or passenger antigens, prepared with hybrid-DNA techniques, may be added during the preparation procedure. This additive may be put in at any step in the procedure, as is described in EP 0 109 942 B1 particularly on pp. 4-8. The preferred method is to put in the additive before adding lipids and glycosides.

Furthermore, the base to be used may be antigens and mucus targeting molecules or passenger antigens that are more or less purified, synthetic or prepared with hybrid-DNA techniques and then one nay proceed as is stated in EP 0 109 942 B1 on pp. 4-8. Here it may be appropriate to add lipids before the complex has been isolated and purified, as is described in the applicant's European patent EP 0 424 380 B1.

The lipids used are particularly those described in the applicant's patent EP 0 109 942 B1 in particular on p. 3 and in patent EP 0 436 620 B1 on p. 7 lines 7-24. Especially sterols such as cholesterol and phospholipids such as phosphatidylethanolamin and phosphatidylcolin are used.

The lipids may also include lipid-containing substances that bind to the cell-binding components, such as glycolipids including the cholera toxin's receptor which is the ganglioside GM1 and fucosed blood group antigen. The cell-binding components can then function as mucus targeting molecule and be bound to the lipid-containing substances through simply mixing them with complexes that contain them.

It is also possible to first prepare iscom particles with one antigen and couple on another antigen using previously known coupling methods, preferably chemical coupling methods, as is described in the applicant's European patents EP 0 180 564 B1 and EP 0 436 620 B1.

Another method is to use matrix as a base, preparing it by solubilising at least one sterol in a solvent, adding the glycoside or the saponines and the other lipids, after which the solubilising agent may be removed if it is not acceptable in the finished product. Matrix is usually transferred to a water solution in which the individual parts are not dissolvable. The solvent can be removed, for example, by gel filtration, ultra filtration, dialysis or electrophoresis. The matrices may then be purified from an excess of sterol and saponin by means of, for example, centrifugation with a density gradient or by gel filtration.

The solubilising agent may be any of those mentioned in EP 0 436 629 B1, p. 5, lines 24-45. The other components and the preparation process are also described in this document.

Passenger and mucus targeting molecule can be coupled to matrix using current coupling methods, see above. The preferred method is to mix the antigens with matrix before administration.

The glycosides used in the preparation product may be those described in EP 0 109 942 B1, p. 4, last paragraph. The preferred method is to use saponins such as triterpensaponins, particularly Quil A or components of it, particularly those described in the applicant's European patent EP 0 436 620 B1 p. 4 lines 19-46. These may be QHA, QHB, QHC or other compositions of Quil A glycosides that are adjuvants. It is also possible to incorporate other adjuvants or immunomodulatory components than the glycosides in the iscoms or in the matrices as mentioned in EP 0 436 620 B1.

It is also feasible to mix the antigen with iscom particles in which an antigen has been integrated or with iscom and/or iscom-matrix to which an antigen has been coupled. Other adjuvants or immunomodulatory components may be mixed with iscom and/or the iscom-matrix and do not need to be integrated into the complexes or coupled with them. Examples of such adjuvants are provided in Cox et al., CRS, 1992. The preferred method is to use MDP, MTP and avridin. It is also possible to mix both transport molecule and passenger antigen with an iscom complex or matrix, in which case the iscom complex contains a different antigen molecule.

If the antigen is lacking hydrophobic or amphiphatic groups, these can be added on so that the antigen will be able to bind to the iscom particle. Examples of such groups are to be found in EP 0 242 380 B1 p. 9 and in EP 0 436 620 B1 p 6 line 33 to p 7 line 6, where the coupling methods are described.

The relative amounts of cholesterol, lipids and antigen that can be used are seen in the above-mentioned patents EP0109942B1, EP0 180 564 B1, EP 0242 380 B1 and EP0436620B1

In making matrix the ratio of sterol, other lipid, and glycoside is 0.2-10:0.2-10:1-100, preferably 1:1:5.

In principle, the components can be put in at any ratio whatsoever. It has been shown that the finished product receives the weight ratio between the various components as given above, and that the excess does not enter in. If too much of the other lipid is used, the complex becomes fatty and week and crumbles easily. Too little of the other lipid leads to the complexes not being formed and annullar ring-shaped subentities being formed instead. This can be determined through electron microscopy.

It is possible to determine whether iscom or matrix has been obtained by examining the product in an electron microscope.

Typical matrices or iscoms have a characterically open, spheric structure consisting of circular subunits or parts of the spheric structure, as can be seen in fig. 3 in EP 0 109 942 B1. The iscoms have a lower sedimentation constant than corresponding micelles and often a higher sedimentation constant than the corresponding monomeric forms of protein or peptide. Matrices and iscoms have a sedimentation constant of approximately 12-22 S, in particular 20 S.

The weight ratio of sterol, other lipid, protein and glycoside is 0.2-10:0.2-10:0.2-10: 1-100, preferably 1:1:1:5-10 in subcutanous administration. In oral or intranasal administration, the amount of glycoside may be higher in the ratio above, namely 1-200, preferably 5-20.

These are the appropriate amounts both when matrix is first produced and bound to the antigens using chemical coupling methods and later when iscom particles are being made.

Iscom or iscom matrix can be prepared in compositions containing a solubilizing agent,
e g water or sodium chloride. The composition may also contain the detergent used in making the complex as a solvent, if it is acceptable from the point of view of human or veterinary medical practice. Moreover, the compositions may contain other additives and filler agents if they are acceptable to human or veterinary medical practice.

Such a composition may contain, for example, an iscom complex and an inert filler such as sodium chloride. It may also consist of a matrix mixed with antigen.

The vaccine may be made available in modes of administration that contain one unit with matrix in a composition containing an inert filler and an unit with the antigen in a composition containing an inert filler. These two compositions are intended to be administered at the same time.

By incorporating Heq 2 antigen into iscoms, an unexpected immune response has been evoked in horses after vaccination of foals even at as low an age as two weeks i.e. in animals with an immature immune system and when high maternal antibody titers ocurring. The vaccination program comprised two intramuscular injections at a two week interval. During the following natural infection, the vaccinated foals were protected while the non-vaccinated foals were not (Nordengran, A., Rusvai, M., Merza, M., Ekström, J., Morein, B. Belak, S. Vet. Microbiol. 51, 55-68, 1996, Example 1). This is the first time a successful vaccination has been carried out when a horse has an immature immune system and in presence of maternal immunity.

Similarly, by incorporating canine distemper antigens in iscoms newborn seals responded immunologically. Three weeks old puppies responded to parvovirus vaccination with a virus-iscom matrix formulation and one week old lambs responded immunologically with a significant protective immunity to oral administration with a rotavirus - iscom-matrix formulation. Two days old mice responded immunologically to Sendaivirus antigens incorporated in iscoms.

The amount of iscom, matrix and antigen is chosen to be pharmaceutically effective and can be estimated by the man of art.

Iscom or iscom matrix can be prepared in compositions containing a solubilising agent, e.g. water or sodium chloride. The composition may also contain the detergent used in making the complex as a solubilising agent, if it is acceptable from the point of view of human or veterinary medical practice. Moreover, the compositions may contain other additives and filler agents acceptable to human or veterinary medical practice.

Such a composition may contain, for example, an iscom complex and an inert filler such as sodium chloride. It may also consist of a matrix mixed with antigen.

The vaccine may be made available for modes of administration that contain an entity with matrix in a composition containing an inert filler and an entity with the antigen in a composition containing an inert filler. These two compositions are intended to be administered at the same time.

### EXAMPLE 1

Equint Herpes virus Type 2 (HEV-2) is a lymphoproliferative virus that infects horses of all ages and can be isolated from the horses' white blood cells and especially lymphocytes. In young foals, for example during the first month after birth, the virus evokes first a mild illness in the respiratory passages in a so-called viral phase. The foal seems to recover but after a period of 2-3 weeks, it is taken ill again. At this point, a complication arises in the form of a secondary infection with bacteria, above all Rhodococcus equi which gives rise to lung abscesses (the bacterial phase). Serum treatment has to a certain extent given protection, while vaccinations using conventional vaccines have had no effect. A partial reason for this is that the foal does not develop an antibody response after vaccination. It is well known that animals and humans during the period after birth when they have maternal antibodies do not develop immunity after vaccination. In the following example we show that the foals who are vaccinated with iscom vaccine develop an immune response that can be measured as an antibody response and that gives protection against natural infection.

### MATERIAL AND METHODS

### Virus

A Hungarian isolate of EHV-2 (stem KT-5797) was used. The virus was cultivated in a rabbit cell line (RK-13). The cells were cultivated in Eagle's minimal essential medium (EMEM) with Earle's salts supplemented with a 10% fetal calf serum, 100 IE of penicillin and 100 mg streptocymin/ml. Cells and cell debris were removed by centrifugation at 2,000 g for 20 minutes at 4ºC. The top liquid was concentrated 10 times through ultrafiltration (Filtron, Clinton, MA, USA) with a exclusion limit of c. 100 kDa. Concentrate of virus was sedimented at 14,000 rpm for 90 minutes at 4ºC in a Kontron TST-28 rotor. The sediment was dissolved in 500 ml of phosphate buffer, sodium chloride (PBS), pH 7.4, was layered on a metricamid gradient of 10-50% (Nyegaard & Co., Oslo, Norway) and was centrifuged in a Kontron TST-41 rotor at 14,000 rpm for 16 hours at 4ºC. The virus band was collected and specimens were taken for electron microscopy and protein determination.

### PREPARATION OF ISCOMS

Purified virus was dissolved in a non-ionic detergent -1-0-n-octylglycopyranoside (OG) (Boehringer, GmbH, Mannehim, Germany) in a concentration of 2% for one hour during vigorous shaking. Solubilised virus was layered on a discontinuous sucrose gradient consisting of 2 ml sucrose of 20% containing 0.5% OG layered over a sucrose cushion of 30%. After centrifugating at 40,000 rpm in a Kontron TST-41 rotor for 45 minutes at 4ºC, the sample volume plus the layer of the sucrose of 20% were collected. Then Quil A (Spikoside, Iscotec, Luleå, Sweden) was added to a final concentration of 1%. The mixture was dialyzed for 72 hours against 0.1 M ammonium acetate buffer pH 7.0 at 4ºC. The resulting iscom preparation was centrifuged through sucrose of 10% at 40,000 rpm in a Kontron TST-41 rotor for 16 hours at 4ºC.

The pellet that contained iscoms was dissolved in 1 ml PBS, pH 7.4.

### VIRUS neutralization (VN) TEST

Serum dilutions (two-steps dilutions) were incubated with 10 TCID50 of virus at 37ºC in microtiter plates with four wells per dilution. The mixture was inoculated at 37ºC and was controlled daily for a cytopathic effect (CPE). The test was read when complete CPE had developed in control tubes. The titer was calculated as final dilution of serum which elicited complete neutralization.

SDS-Polyacrylamidgelelectrophoresis (Nordengran, A., Rusvai, M., Merza, M., Ekstrom, J., Morein, B. Belak, S. Vet. Microbiol. 51, 55-68, 1996) was carried out to establish the polypeptide bands that formed the iscoms.

Electron microscopy was carried out to establish iscom formation.

### EXPERIMENTAL DESIGN

### Experiment Year 1

Each dose contained 40 µg. The first dose was given at two weeks of age.
Three foals were immunized once.
Three foals were immunized twice intramuscularly (i.m.) with two weeks' interval.
Three foals were immunized three times with two weeks' interval.

Eight foals were unvaccinated controls (Table 2).

### Experiment Year 2

19 foals were vaccinated first i.m. at two weeks of age with 40 µg Heq2 iscoms. The second immunization i.m. with 40 µg occurred two weeks later.

Five foals were unvaccinated controls (Table 2).

**Collection of specimens.** Serum for testing were taken from foals at the time for the first vaccination, and every week after that. Serum from unvaccinated animals were taken every 3 to 4 weeks.

### RESULTS

Iscoms are characterised as described in the manuscript (Nordengran, A., Rusvai, M., Merza, M., Ekström, J., Morein, B. Belak, S. Vet. Microbiol. 51, 55-68, 1996).

### Antibody response in foals with preexisting maternal antibodies (reciprocal titers)

Foals with preexisting VN-antibody titers of 2 or 4 all responded with significant antibody increases of 2-fold or more (Table 1). Of the six foals with preexisting titers of 8, three had a twofold or higher increase while two foals had an increase of one-fold and one animal after both the first and the second immunization showed a titer of 8. One foal with a titer of 16 increased its serum titer to 1:32 after the first immunization but later the titer decreased to 8. Foals with serum titers of 32 or 64 increased their titers 1- or 2-fold.

### ISCOM VACCINE EVOKES PROTECTION AGAINST NATURAL INFECTION

### Experiment 1 - virus neutralizing serum titers

Foals were vaccinated one, two and three times and thereafter left to be exposed to the natural infection spread that had existed in the horse farm for many years. All the vaccinated animals remained free of symptoms except one animal who had been immunized once and who showed mild respiratory symptoms and a slight rise in temperature.

Non-vaccinated foals (three) all showed severe respiratory symptoms and one died (Table 2).

### Experiment 2 - second year

24 foals took part in the experiment. Five were kept as unvaccinated controls. The other foals were vaccinated for the first time at two weeks of age and the second time two weeks later. The animals were thereafter left to be exposed to the natural infection spread that had existed in the horse farm for many years.

18 out of 19 vaccinated animals remained healthy and free of symptoms. One animal showed a rise in temperature for one week and symptoms.

Four out of five unvaccinated foals had severe respiratory symptoms and prolonged fever and three of these foals died. One foal remained free of symptoms.

### CONCLUSION

In spite of the fact that the foals who were vaccinated had maternal antibodies, two vaccinations were enough to elicit clearcut antibody increases, which were not found in the unvaccinated control animals. Vaccinated animals were protected against natural infection when the vaccination was given at least twice.

The experiments show that an iscom vaccine is effective even when the animals are very young with immature immune system and when maternal antibodies are preexistent.

### EXAMPLE 2

Vaccination with the measles virus (MV) with a live virus are carried out around the world, generally with good results in the western world, while the results in the third world are disappointing since the virus causes high mortality even among the vaccinated population. A serious problem in this regard is that children in these countries are infected at an age when they still have maternal immunity that blocks the effect of the vaccination. The following experiment was performed to show that iscom vaccine evokes antibody response in monkeys that have experimentally been furnished with antibodies from hyperimmunized monkeys.

### METHODS

**Passive transmission** of MV-specific antibodies. To attain monkeys with a predetermined amount of specific virus neutralizing (VN) antibodies at the time of immunization, different volumes of hyperimmune sera pooled from 16 monkeys that were previously infected with a wild strain of MV (MV-BIL) were transferred intravenously to monkeys with no MV antibodies (i e naïve monkeys) 48 hours before immunization.

**Preparation of vaccine.** Attenuated Schwartz measles vaccine was a gift from Institut Mérieux, Lyon, France (103 50% tissue culture doses [TCID50] was used per dose and per ampule). MV-iscoms were prepared according to the centrifugation method described by Morein, B., Sundquist, B., Höglund, S., Dalsgaard, K, and Osterhaus, A. Nature 308, 457-460, 1984. Short MV was dissolved in 2% Triton X-100 and was dispatched on a sucrose gradient of 20-60% containing 2% Quil A (Spikoside, Iscotec, Luleå, Sweden) and was centrifuged in a SW28 rotor at 20,000 rpm for 18 hours at 4ºC. The gradient fraction that contained iscom particles was pooled, dialysed and analyzed by electron microscopy, SDS-polyacryladmidgelelectrophoresis and ELISA for quantifying of H and F proteins in the iscom particles.

**Vaccination of cynomolgus monkeys.** Two-year-old MV-negative monkeys were immunized with MV-Schwartz (4 monkeys with 100 TCID50 per dose) or intramuscularly twice with a four weeks' interval or with iscoms (4 monkeys with 10 µg antigen per dose). Blood tests were taken every week for analyses of serum antibodies using ELISA and virus neutralizing tests.

**Serological tests.** Plasma was extracted from the heparin-treated blood tests. The plasma was heat-inactivated for 30 minutes at 56ºC. Antibodies specific to MV were tested in the IgM and IgG classes. IgM ELISA titers were expressed as optic density (OD) values at 450 nm. IgG ELISA titers were expressed as reciprocal dilution of individual sera that cause a 50% inhibition of maximal OD-values at 450 nm. VN-antibody titers were expressed as reciprocal dilution of sera that inhibits cytopathic effect in Vero cells that are infected with 100 TCID50 of MV-Edmonston virus strain.

### RESULTS

### Virus propagation and serum antibody response after MV-Schwartz vaccination in the presence of MV-antibodies that have been transferred passively

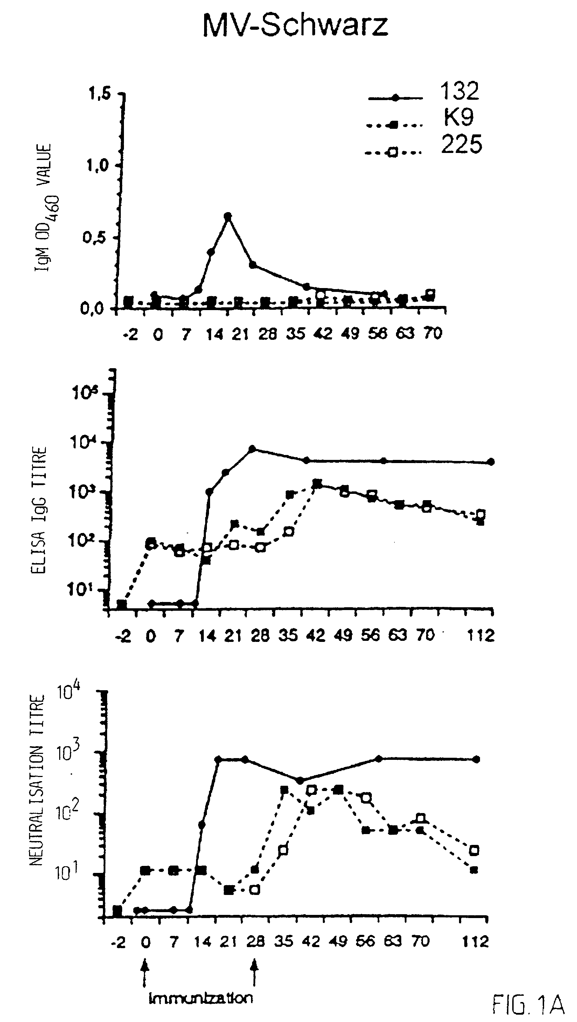

Before vaccination, the monkeys were inoculated with various amounts of serum containing antibodies against MV (see Methods). In the absence or presence of low titers of passively transferred MV-specific antibodies (VN-titer<5), the monkeys developed a marked viremia 7-9 days after immunization with MV-Schwartz. The monkeys also developed specific serum antibodies against MV of IgM and IgG classes. Monkeys with serum antibody titers >5 showed no signs of virus propagation and the specific IgM and IgG titers, like the VN-titers, were greatly reduced in monkeys (K9 and 225) who had passive antibody titers at the time of the vaccination as compared to monkey 132 who had no marked titers at vaccination (see Fig. 1A). These data show that the presence of low MV-specific serum antibodies (>5 (or 0.08 IU/ml)) interferes with the replication of MV-Schwartz and with the development of an MV-specific antibody response.

### Vaccination using an iscom-based measles vaccine

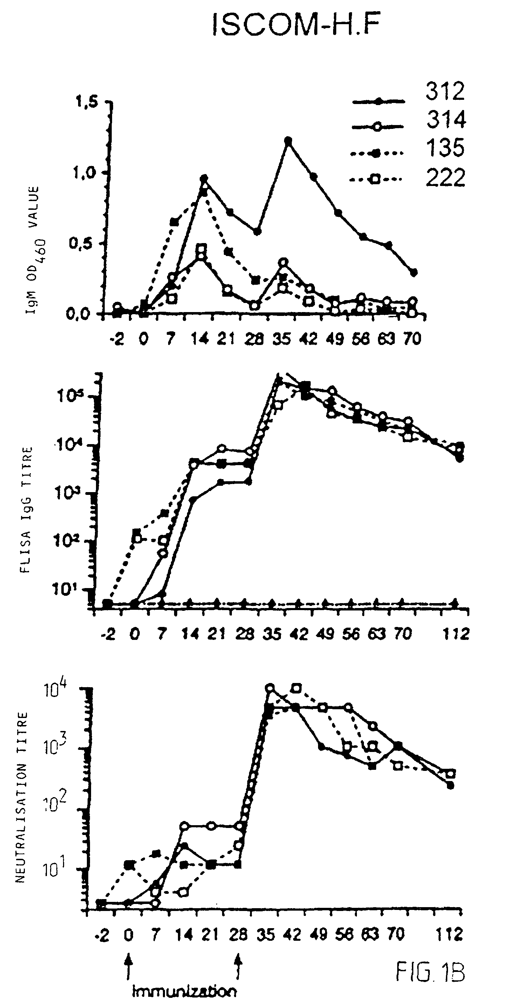

After the immunizations using iscoms, MV-seronegative monkeys (312 and 314) and monkeys who were serum-positive through passive transferred of MV-antibodies (135 and 222) responded with clear IgM antibody response, in contrast to MV-Schwartz-immunized monkeys. Iscom vaccine evoked high IgG serum antibody responses which were a hundredfold higher than the response from MV-Schwartz-immunized monkeys. All of the monkeys, both serum-positive and serum-negative, responded with virus neutralizing antibodies of the same magnitude (Fig. 1A, 1B).

### CONCLUSION

The results of this experiment show that iscom vaccines induce immune response in the presence of passively transferred specific antibodies and therefore support the results from Example 1, showing that iscom vaccines are effective when passively transferred antibodies are preexistent. The monkeys in this experiment were immunocompetent.

Fig. 1B shows that measles virus (MV) iscoms (monkeys 312, 314, 135 and 222) evoke serum antibodies against MV of IgM, IgG class, which neutralize viruses (VN). MV-Schwartz vaccine does not have this capacity; MV-specific IgM antibodies are not evoked at all or a very weak response is evoked that is transient and passes quickly. IgG and VN-antibody responses are 10 to 100 times lower than those evoked using iscom vaccine.

### EXAMPLE 3 (Vaccine)

Canine distemper virus (CDV) is one of several viruses causing disease in young puppies and through the years it has been a major problem in kennels. Efficient vaccines are available for adults, but no vaccine is available which is efficient in the juvenile period. No vaccine is guaranteed to be effective before the age of 12 weeks. In kennels CDV and also canine parvovirus may cause high moribidity and mortality before the age they respond to vaccination depending I. on an immature immune system i.e. an immune system which is not competent as in adults (Ridge, J.P., Fuchs, E.J., Matzinger, P. Science 271, 1723-26, 1966; Billingham, R.E., Brent, L., Medawar, P.B. Nature 172, 603, 1953; Bandeira, A., Coutinho, A., Carnaud, C. Jacquemart, G., Forni, L. Proc. Natl. Acad. Sci. USA, 86, 272, 1989; Schurmans et al., J. Immunol. 145, 2465, 1990). II the maternal immunity mainly antibodies transferred from the mother to the newborn exerts a blocking effect on the vaccine.

In the present study newborn pups from seals, between 1 and 8 days old, were selected to have maternal antibodies (group 1) measured by virus neutralization (VN) test.

Seals in Group II were not selected with regard to the presence of maternal immunity, thus containing both seropositive and seronegative pups with regard to CDV.

In the North sea, in the Baltic and around the coasts of Great Britain, many seals died because of outbreaks of canine distemper-like disease caused by a related virus in the morbillivirus group. For that reason a vaccine concept based on the iscom technology was tested. Live CDV-vaccires cannot be allowed to wild animals because of the risk to cause diseases and to cause an epizootic among wild animals.

Propagation of virus was done in VERO-cells. The virus was purified by ultracentrifugation as described for Heq2 virus example 1. The iscoms were prepared as described for Heq 2 virus (Example 1).

### Immunization

The pups were immunized intramuscularly (i.m.) at an age of one to eight days. Eight days after immunization serum samples were collected and tested in the VN-test (Table 4 and 5).

In group I, Table 4 encompassing unselected seals from the sea outside the coast of Wales were immunized i.n. with 7 µg antigen per animal. Eight days later, serum samples were collected. In this group eight out of the 13 serum from the seals showed increased HI titers. Prior to vaccination, five of these sera had no detectable maternal antibodies and these animals responded to the vaccination, which also animals with maternal antibodies did. No animals showed decreasing titres which would be expected if no immune response was achieved.

In group II (Table 5) 23 pups were selected to have maternal antibodies. Nine sera of these animals showed increased HI-titres, 12 sera had the same antibody titres after vaccination as before and only two animals showed decreased VN-titres.

In general the VN-titres should have decreased considering the half life time of 12 days for seal antibodies. The results clearly show that the majority of the seals had responded to the vaccination.

During the past years, more than 200 seal pups have been vaccinated with a CDV iscom preparation upon arrival in a seal sanctuary in The Netherlands. The age of these animals ranged from 0-14 days. Apart from a rise in CDV neutralizing antibodies which in the majority of the cases also ocurred in the presence of maternal antibodies, no cases of phocine distemper could be demonstrated in any of these animals of which more than 90% were successfully rehabilitated and reintroduced into their natural habitat three to four months after admission. This in spite of the fact that we have shown that the PDV infection was still spreading in the wild population (Visser et al., Vet. Rec. 133, 320-322, 1993) and in the sancturary from time to time, without causing significant clinical symptoms in the sanctuary. From some of the animals in the sanctuary showing a mild conjunctivitis, PDV could be isolated from time to time.

### CONCLUSION

This experiment clearly shows that juvenile seals respond to vaccination in the presence of or without maternal antibodies. Such vaccines are highly desired both for man and animals.

### EXAMPLE 4

### Protective mucosal immunity induced in lambs by oral administration of inactivated rotavirus adjuvanted with iscom-matrix

### Introduction

Rotaviruses are recognized as the major cause of severe diarrhoeal disease in children younger than 5 years of age in both developed and developing countries, resulting in some 870,000 deaths and several million cases of severe diarrhoea in this age group annually. They are of similar significance as a cause of neonatal dirrhoea in many domesticated animal species. With the exception of a study describing the primary mucosal immune response in rabbits, there has been little detailed characterisation of this response.
In this example, we examine the hypothesis that a mucosal immune response as well as a systemic response can be induced following oral immunization with inactivated bovine rotavirus and iscom-matrix which would result in a protective immune response to subsequent live virulent virus challenge. It should also be emphasized that the lambs in this experiment (6 days old) are very young and that they are in an age when they are less immunocompetent than adults (Ridge, J.P., Fuchs, E.J., Matzinger, P. Science 271, 1723-26, 1966; Billingham, R.E., Brent, L., Medawar, P.B. Nature 172, 603, 1953; Bandeira, A., coutinho, A., Carnaud, C. Jacquemart, F, Forni, L. Proc. Natl. Acad. Sci. USA, 86, 272, 1989; Schurmans et al., J. Immunol 145, 2465, 1990) and during a period the maternal antibodies are expected to prevail which makes the successful vaccination even more difficult. For that reason it would be even more interesting to explore whether the combination of an oral mucosal administration with a modern adjuvant system will overcome the problem of neonatal immunization.

### MATERIAL AND METHODS

### Viruses

The vaccine virus was a bovine rotavirus strain, UK, grown in MA 104 cells. The infecitivity titre, before inactivation was 106.8 fluorescent focus units (ffu)/ml, and after treatment with a 5% (v/v) of a 0.1 M binary ethylene imine (BEI) solution at 37°C for 24 hours, no infectivity was detected. Virulent lamb rotavirus strain K923 passaged in gnotobiotic lambs was used for challenge. Each lamb received 108.5 ffu suspended in 5 mls of sterile PBS.

### Experimental design

Lambs were born and maintained in gnotobiotic isolator units. At 6 days of age, lambs were orally inoculated with a mixture of either PBS and 500 µg of iscom matrices (Advet, Uppsala, Sweden) (PBS/ISC;n=6), inactivated bovine rotavirus (strain UK) and 500µg of iscom matrices (RV/ISC; n=5), or inactivated bovine rotavirus alone (RV; n=3). Lambs were challenged 21 days later with live virulent ovine rotavirus (strain K923) and killed 1-2 weeks after challenge.

### Sample collection and analysis

Blood for serum and nasal secretions were collected at initial immunization and then at regular intervals to determine the levels of specific and total IgA and IgG antibodies by ELISAs and neutralising titres by virus neutralisation tests. Faecal samples were collected daily after immunization and challenge until rotavirus excretion was no longer detected. Rotavirus excretion was assayed by detection of double-stranded RNA by sodium dodecyl sulphate-polyacrylamide gel electrophoresis (SDS-PAGE). PBLs were collected at initial immunization and then at weekly intervals to determine the numbers of specific IgA and IgG secreting cells by ELISPOTs and the distribution of lymphocyte subpopulations by FACS analysis. Lambs were killed 1-2 weeks after challenge and lymphocyte populations isolated from Jejunum Peyers patches (JPPs), Ilium Peyers patches (IPPs), Mesenterial lymphnodes (MLNs) and small intestine Intestitiel epethelial lymphocytes and Lamina propria lymphocytes (IEL and LPL). Numbers of specific IgA and IgG producing cells in JPPs, MLNs and LPLs and were determined by ELISPOT. Lymphocyte subpopulations were analysed by FACS. Cytokine expression (γ-IFN, IL-2, and IL-4) in MLNs and JPPs was visualized by RT-PCR and hybridisation. The levels of specific and total IgA and IgG antibodies and neutralisation titres in intestinal secretions were determined using intestinal mucus.

### Lymphocyte isolation

Peripheral blood lymphocytes were collected an processed as previously (Puri, N.K., MacKay, C.R., Brandon, M.R. Immunology 55, 725-33, 1985)described and resuspended at the appropriate concentration in complete IMDM containing 1mM glutamine, 100 units/ml penicilling, 0.1 mg/ml streptomycin, 2 µg/ml amphotericin B (Sigma, St Louis, USA) and processed as previously described. The lymphocytes were finally suspended at the appropriate concentrations in complete IMDM. Pieces of small intestine were collected and processed with minor modifications of previously described methods. Small intestine was cut into 10-cm segments, inverted, washed three times at 37°C with gentle stirring in HBSS without calcium and magnesium containing 2 mM EDTA to free epithelial cells and intraepithelial lymphocytes. The tissues were washed in RPMI 1640 and placed in RPMI containing 1mM glutamine, 100 units/ml penicillin, 0.1 mg/ml streptomycin, 2µg/ml amphotericin B, 5µg/ml gentamycin, 80 units/ml collagense XI (Sigma), 0.1 mg/ml deoxyribonuclease type V (Sigma), and 10% FCS at 37 °C to allow the lamina propria lymphocytes to be removed. The IEL and LPL suspensions were passed through sterile glasswool columns, layered onto an equal volume of Lymphoprep® and spun down. Lymphocytes were harvested at the interface and washed twice in HBSS containing 1 mM glutamine 100 unit/ml penicillin, 0.1 mg/nl streptomycin, 2 µg/ml amphotericin B, and 2% FCS before being suspended at the appropriate concentration in complete IMDM.

### Assays

**Virus neutralising assays.** Virus neutralising assays for bovine rotavirus strain UK and lamb rotavirus strain K923 were performed in microtitre plates and the endpoints or virus neutralising titre (VNT) were determined by 60% reduction in fluorescent focus units.

ELISAs. Specific-rotavirus ELISAs utilised microtitre plates, (Nunc, Maxisorb), coated with a rabbit antirotavirus capture serum, lamb RV K923 and uninfected MA 104 extracts as negative antigens binding control, the test and control samples, horseradish peroxidase-conjugated donkey anti-sheep IgG (The Binding Site) or mouse anti-bovine IgA, with the colour developed by adding substrate H2O2 and σ-phenylenediamine dihydrochloride to the bound peroxidase. The reaction was stopped with 2 M H2SO4, and the absorbance measured at 490 nm. The net absorbance was calculated by subtracting the values in the negative wells from the corresponding K923 wells.

Total isotype ELISAs utilised microtitre plates coated with rabbit anti-sheep Igs (DAKO) or pig anti-sheep IgA, test samples, diluent, and control samples, horseradish-peroxidase conjugated rabbit anti-sheep IgG (Pierce) or mouse anti-bovine IgA, with the same colour development as described above. OD values in the diluent wells were substracted from the corresponding test wells. For each ELISA a standard was used: a hyperimmune lamb serum for the IgG concentration of 9,600 mg/L for the total IgG ELISA; a batch of IgA, purified from clarified lung fluid of a sheep infected with an ovine retrovirus (Jaagsiekte) (JSRV) for the total IgA ELISA. Each standard was assayed at eight 2-fold dilutions, and the net absorbance as a function of arbitrary antibody units or mg per ml respectively was fitted to a lin-log sigmoid curve. In each case the resulting standard curve had a fit of r>0.95. The resulting parameters were then used to interpolate antibody units or concentration (mg/ml) for each of the test samples, taking into consideration the dilution factor at which each sample was tested. Specific and total ELISA results were combined and expressed in units/mg of IgG or IgA.

ELISPOTs. Isotypes anti-rotavirus ELISPOTs utilised mocrotitre plates, (Nunc, Maxisorp), coated with a mouse anti rotavirus capture serum, sucrose-purified rotavirus strain UK as antigen at a concentration of 5µg/ml, the PBL suspension (5 x 105 cells/ml), horseradish peroxidase-conjugated rabbit anti-sheep IgG (Pierce) or mouse anti bovine IgA, with the spots made visible by adding substrate H2O2 and 3-amino-9-ethylcarbazole (AEC) to the bound peroxidase. The reaction was stopped, when spots were visible, by flicking off the substrate. Spots were counted (mean of 6 replicates) and expressed as spot forming cells (SFC)/106 lymphocytes.

**FACS analysis.** Lymphocyte subpopulations were determined with a panel of mouse monoclonals specific for ovine CD4 (17D) (MacKay, C.R., Hein, W.R., Brown, M.H., Matzinger, P. Eur. J. Immunol. 18, 1681-8, 1988; Maddox, J.F., MacKay, C.R., Brandon, M.R. Immunology 55, 739-48, 1985), CD8 (7C2)(Maddox, J.F., MacKay, C.R., Brandon, M.R. Immunology 55, 739-48, 1985), γδTCR (86D), light chain (VPM8)(Puri, N.K., MacKay, C.R., Brandon, M.R. Immunology 55, 725-33, 1985), and CD45R (73B)(MacKay, C.R. Marston, W.L., Dudler, L. J. Exp. Med. 171, 801.17, 1990). A second antibody, fluorescein-labelled rabbit anti-mouse IgG (DAKO), was applied and cells were scanned and counted with a FACScan® (Becton-Dickinson Ltd).

### RESULTS

### After vaccination

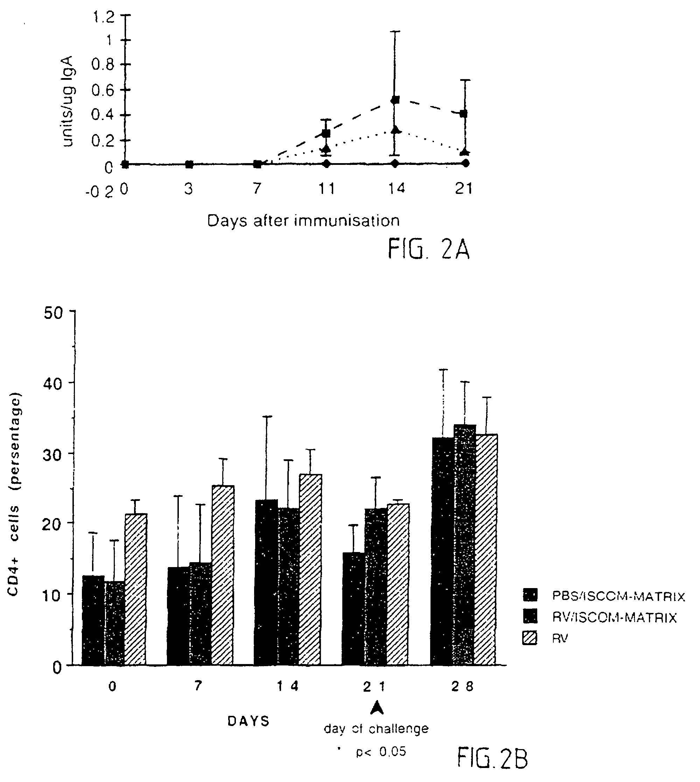

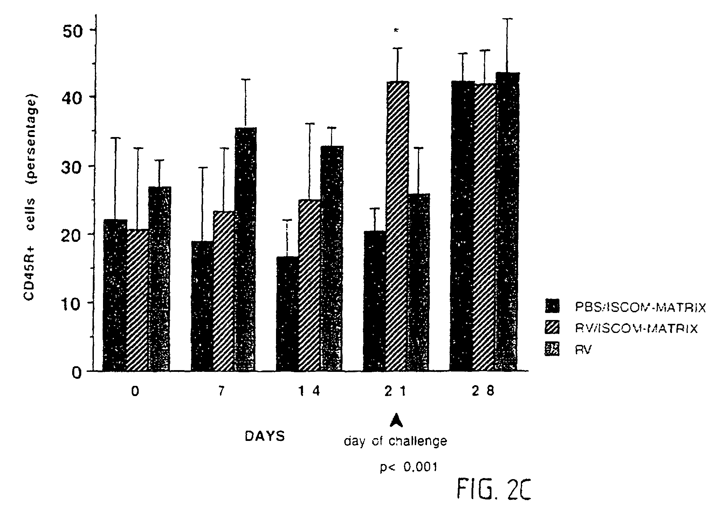

The levels of specific IgG antibodies in blood after immunization was higher for the animals vaccinated with RV iscom matrix (Fig. 2 A). Significantly higher percentages of CD4+ cells at 21 days after immunization in both RV-vaccinated groups (Fig. 2 B). CD45R+ cells (Fig. 2 C) were only recorded in the RV/ISC-matrix vaccinated group in blood most important from the point of view that these cells are memory cells and constitute a basis for a fast response for a subsequent infection with rotavirus. Interestingly, the PBS/ISC-matrix, i.e. no-antigen group showed no increase of CD4+ cells but did show a significant higher percentage of γδTCR-cells (not shown).

### After challenge infection

It is expected that after challenge infection animals respond immunologically. However, a solitary immunization orally has not before, with non-live (non-replicating) rotavirus formulation, induced protection to infection or to disease. The protection to disease is dependent on I. the existing immune defence II on the readiness of the immune system to respond fast against the infecting agent in this case RV. This readiness is dependent on memory cells i.e. the CD45R+ cells which were induced by RV iscom-matrix which were detected day 21 after vaccination and before challenge infection (Fig. 2 C). The read-out of the readiness against infection is the excretion of the infectious agent in this case the RV. It should be noticed that this experiment is the first of this kind in a ruminant with very complicated alimentary system. Therefore, to reach optimal conditions with regard to dose of adjuvant (iscom-matrix) and antigen etc. a series of experiments is required. However, the very promising prospect for a successful vaccine based on the iscom and iscom-matrix technology is strongly indicated.

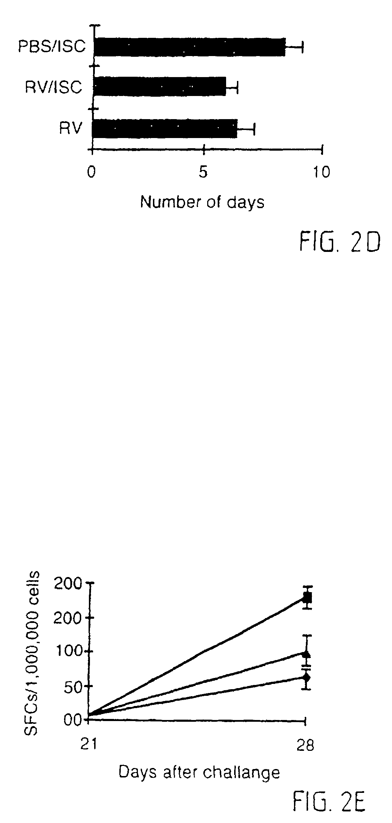

After challenge all lambs excreted rotavirus. Both RV-vaccinated lambs excreted virus for a shorter period than the controls namely 5-6 days, 6-7 days and 8-9 days respectively, however this was only significant (p=0.027) in the RV/ISC-matrix vaccinated group strongly indicating the potential for developing an effective vaccine (Fig. 2 d).

As expected, all lambs had after challenge specific IgA and IgG antibodies in serum, nasal secretions, and gut scrapings and antibody secreting cells in blood and gut associated lymphoid tissues (MLNs and JPPs) after challenge.

Notably, the RV/ISC-matrix vaccinated groups had significantly increased numbers of specific IgA (Fig. 14 E) and IgG producing cells in blood indicating a prining effect of the vaccination. The priming effect is dependent on the development of memory cells. The numbers of specific IgG producing cells was also significantly higher compared to the non-adjuvanted RV-vaccinated group. After challenge, no differences were found in specific IgA (Fig. 2 E) or IgG producing cells in the GALT between the three groups. The specific IgG response was similar to the specific IgA response.

Neutralizing antibodies against UK and K923 were detected in all groups in serum. After challenge, all lambs had an increase in CD4+ cells and in the PBS/iscom-matrix and in the RV-vaccinated groups the CD45R+ cells increased to a similar level as observed in the RV/ISC-vaccinated group before challenge.

### DISCUSSION

It is surprising that a single oral dose of inactivated non-replicating rotavirus adjuvanted with iscom-matrix resulted in a significantly reduced period of virus excretion upon subsequent challenge with live replicating virus. A significantly reduced period of virus excretion was only observed in the RV/ISC-vaccinated group and not in the RV-vaccinated groupd, although the small number of lambs in this group limits the statistical analysis. Characteristics of the immune response of primed animals to virus challenge included increased levels of specific IgA and IgG antibodies in nasal and intestinal secretions and, in the RV/ISC-vaccinated group, increased numbers of circulating specific IgA and IgG producing cells.

It is also surprising that RV-adjuvanted with iscom-matrix with only one dose would prime an immune response and significantly reduce the infectious period in view of the results of Mowat (Mowat et al., Immunology 72, 317-322, 1991) showing in mice that several immunisations and high doses were required to induce IgA response following oral administration of OVA.

### CONCLUSION

It is unexpected that a clear-cut priming would be achieved by oral administration of one dose of Rotavirus adjuvanted with iscom-matrix or any non-replicating vaccine in view of the young age of the animals i.e. one week when they still are in the neonatal period and not immunocompetent as adults and also in view of the difficulty to induce immune response with one immunization by the oral route. This is particularly difficult in view of the complicated alimentary system of ruminants. It is likely that the rotavirus or parts of the rotavirus in this respect is suitable for use as a mucosal target seeking device suitable to facilitate the immunization with other antigens as well in the iscom and isocm-matrix formulations. These results implies the possibility to successfully immunise the animals via mucosal immunization during the neonatal period when maternal antibodies are present. The increased number of CD45R+ cells induced by RV/iscom-matrix indicate an increased number of memory cell which can rapidly be recruited by an infection explaning the shortened period for virus excretion following oral challenge and thereby eliminating or decreasing the risk for development of disease.

### EXAMPLE 5

Sendai virus is a paramyxovirus infecting the respiratory tract of mice and causes pneumonia. In this experiment the envelope proteins were extracted with detergent from the virus particles and reassembled into iscoms or into micelles. Both particles are spherical with a diameter of about 40 nm well exposing the antigens. The micelles lack an adjuvant component consisting of envelope proteins while the iscom besides the envelope proteins contains an in-built adjuvant of triterpenoid molecules. The purpose of the experiment is to explore whether Sendaivirus envelope proteins integrated into iscoms induce immune response in two days old mice lacking immunological competence due to an immature immune system. Clasically, a comparatively high dose of 1 µg should induce tolerance at the age of 2 days. The second vaccination in immunologically adult mice was carried out day 42. The readout of the experiment would be a strong immune response after the second immunization in the case the 2 days old mice respond with memory cells after the first immunisation. Mice being primed as immunologically adults would be expected to respond with a low antibody response. In the case tolerance is induced, the mice will not respond to the second immunization carried out day 42.

### MATERIALS AND METHODS

### Virus and iscom formation

The Sendaivirus was propagated in eggs, purified by ultracentrifugation as described for influenzavirus in Patent PCT/SE97 example 3 (priority from SE 9600647-3).

The virus was purified by sucrose gradient ultracentrifugation as described for influenza virus, solubilized with octylglycoside instead for MEGA and iscoms were prepared as described in example 3 Patent PCT/SE97 (priority from SE 9600647-3).

Micelles were prepared as described for iscoms but except for the omission the Quillaja components were omitted resulting in an assembly of the amphipathic envelope proteins by hydrophobic interactions by their transmembrane region to form micelles. (Lövgren, K., Kåberg, H., Morein, B. Clin. Exp. Immunol. 82, 435-439, 1990).

### Experimental design

Groups of eight two-day-old mice as described in Table 6 were immunized subcutaneously (s.c.) with 1 µg or 0.2 µg of influenza virus antigens in iscoms or micelles and subsequently they received the same dose as immunologically adults day 42. Other groups of mice received 1 or 0.2 µg iscoms or micelles day 42. The antibody responses were measured in ELISA as described in Example 4 Pat. XXXXX except that the antigen adsorbed to the plastic plates were iscom with Sendaivirus antigen at a concentration of 0.1 µg/ml. The bleedings to obtain serum were carried out two weeks after the second immunization.

### RESULTS

Mice primed with iscoms at the age of two days and subsequently boostered at the age of 42 days responded with clearcut antibody responses after booster to Sendaivirus antigen. The low dose of 0.2 µg resulted in higher titres Table 6 with ELISA reading values at dilution 1:2000 at OD45 of 2.5 while the higher dose of 1 µg iscoms induced serum antibody response measured as a reading value of 1.1 (OD450).

The mice primed with 0.2 µg iscoms as immunologically adjults responded with low reading values at a dulution 1:1000. The ELISA used is one routinely used to screen mice for serum antibodies to Sendaivirus at the National Veterinary Institute in Uppsala.

None of the mice immunized with micelles either with the low dose i.e. 0.2 µg or the higher dose of 1 µg responded to the immunization nor after one or two immunizations. This may indicate a tolerance induction by the primary at a low age i.e. when the immune system is immature. In contrast, the same doses of antigen in an iscom induced clearcut serum antibody responses that were primed in immunologically immature mcie which did not develop tolerance but a strong immune response.

### CONCLUSION

Mice immunized (primed) with Sendai iscoms at an age, when they are not immunologically mature and at an age when they are known to respond to vaccination with **a tolerance response** did not do that when primed with Sendaivirus iscoms. But mice immunized with the same doses of Sendaivirus in a non-adjuvanted micelle formation did not respond serologically after two immunization indicating the development of an immunological tolerance to Sendaivirus. This is unexpected that an adjuvant breaks or overcomes the neonatal immunological tolerance.

### EXAMPLE 6

Canine parvovirus cause disease in young puppies and has been a major problem in many kennels. Live and killed vacines are available for adults, but no vaccine is available which is efficient for puppies under the age of 10 to 12 weeks. In a limited study, we show that the parvovirus killed with β-propiolactone and adjuvanted with iscom-matrix is considerably more efficient than a commercial, killed vaccine when used at a lower age of puppies than recommended by manufacturers.

### MATERIALS AND METHODS

### Preparation and purification of virus

The canine parvovirus strain CPV-916 kindly provided by C.G. Sjösten, Pherovet, Malmö was grown in feline lung cells. The virus was harvested on day two. Low speed centrifugation (10 min. at 4200 rpim) was used to free the virus from cell rests. The virus was sedimented by ultracentrifugation. The viruspellet was collected in TN buffer and virus was further purified by gradient ultracentrifugation. CsCl gradient, 1.20-1.40 g/cm3, was prepared, mixed with the sample and centrifugated 30,000 rpm for 12 hrs at -20° C. Two distinct bands were established. The suspension was fractionated and each fraction HA-titrated and pooled with HA titres ≥ 224 which was considered typical for parvovirus and this pool was selected for further processing and dialyzed over night at +4° C in TN buffer.

The virus was diluted to final concentration of one mg per ml PBS used as dilutant and stored at -20° C until use.

From the stored material, the experimental vaccine was prepared by dilution of the virus with PBS to contain 7 µg virus per dose in a volume of 1 ml in PBS.

### Hemagglutination inhibition (HI) test

The H test was carried out using erythorcytes from a three-week-old piglet as described by Klingeborn, B and Moreno-López, J. Zbl Vet. Med. B. 27, 483-488, 1980.
**Protein determination:** was carried out according to Bradford, Analyt. Biochem. 72, 248-254, 1976.

### Experimental design

A litter of puppies from the Swedish dog center in Sollefteå was used in the study. Four puppies at the age of three weeks were vaccinated with the parvovirus/iscom-matrix and three puppies were vaccinated with the commercial, killed vaccine the first time at the age of 3 weeks and the second vaccination 23 days later (Table 7), i.e. both vaccinations were carried out before the age when commercial vaccines are recommended. Serum for testing were taken 15 and 28 days after the first immunization and 10 days after the second vaccination (see Table 7).

### RESULTS

After two immunizations the puppies vaccinated with the parvo/iscom-matrix formulation had serum reciprocal HI-titres of 2560 or 5120 to be compared with reciprocal serum HI-titre of 40 to 160 being induced by the commercial vaccine (Table 7).

### CONCLUSION

It is obvious that the parvo/iscom-matrix experimental vaccine int the limited study presented is superior to the commecial, killed parvo-virus in the age groups of puppies tested. The HI-titres obtained with parvo/iscom-matrix experimental vaccine would be very high even if those were obtained in adult dogs or puppies vaccinated at a recommended age of 12 weeks or more. These results are unexpected in view of the fact that no commercial, killed or even less so a live parvovirus vaccine is expected to be effective in the age groups of puppies tested.

### LEGENDS TO FIGURES

**Fig. 1A, 1B** shows that measles virus (MV) iscoms (monkeys 312, 314, 135 and 222) evoke serum antibodies against MV of IgM, IgG class, which neutralize viruses (VN). MV-Schwartz vaccine does not have this capacity

**Fig. 2 A** Specific IgG antibodies in blood after vaccination; Means of groups were analyzed one-way ANOVA. RV-vaccinated group vs PBS-vaccinated group;
■ RV/ISC-matrix-vaccinated group
   RV-vaccinated group

◆ PBS/ISC-matrix vaccinated group

**Fig. 2 B.** The lymphocyte subpopulation of CD4+ cells (percentage) in peripheral blood at increasing time span after oral vaccination with rotavirus (RV) vaccines with and without iscom-matrix adjuvant and subsequent challenge infection day 21 Means of groups were analysed by one-way ANOVA.

**Fig. 2 C.** The lymphocyte subpopulation CD45R+ cells (percentage) in peripheral blood at increasing time span after oral vaccination with rotavirus (RV) vaccines with and without iscom-matrix adjuvant and subsequent challenge infection day 21 Means of groups were analysed by one-way ANOVA.

**Fig. 2 D.** Virus excretion after challenge infection.

**Fig. 2 E.** Specific IgA producing cells in blood after vaccination and subsequent challenge infection day 21.
◆ PBS/iscom-matrix (no RV antigen)

■ RV/iscom-matrix
   RV (inactivated virus)

**Table 1**

| The development of virus neutralization (VN) antibodies in serum in foals in presence of maternal antibodies | | | |
|---|---|---|---|
| Serum antibody titres | | | |
| after | 1 st vaccination | | 2nd vaccination |
| Weeks | 0 | 1 | 4 |
| | | | |
| | | | |
| Non-vaccinated | | | |
| controls | 2 | 2 | 4 |
| | 2 | 2 | 4 |
| | 4 | 2 | 8 |
| | 2 | 2 | 2 |
| | | | |
| One vaccination | 16 | 32 | 64 |
| | 2 | 4 | 8 |
| | 4 | 2 | 8 |
| | 2 | 2 | 2 |
| | | | |
| Two vaccinations | 2 | 32 | 64 |
| | 2 | 2 | 64 |
| | 2 | 8 | 64 |
| | 2 | 4 | 16 |
| | 4 | 2 | 16 |
| | 4 | 4 | 16 |
| | 4 | 4 | 16 |
| | 4 | 8 | 16 |
| | 8 | 8 | 8 |
| | 8 | 4 | 16 |
| | 8 | 8 | 32 |
| | 8 | 16 | 16 |
| | 8 | 16 | - |
| | 8 | 16 | 64 |
| | 16 | 32 | 8 |
| | 32 | 32 | 64 |
| | 64 | 128 | 128 |
| | 64 | 128 | 256 |

**TABLE 2 (Exp. 1)**

| Protection against natural infection was elicited after two immunizations of EHV-2 iscoms into foals | | |
|---|---|---|
| Group | Number of immunized foals | Number of animals ( ) with symptoms and score of symptoms |
| I | 1 | (1) - |
| | | (1) +/- |
| II | 2 | (3) - |
| III | 3 | (3)- |
| IV | non-vaccinated controls | (1) +++ |
| | | (1) ++ |
| | | (1) + |
| | | |
| - No symptoms | | |
| +/- Body temperature over 39.5°C, mild respiratory passage symptoms for less than 6 days | | |
| ++ Recurrent high fevers or prolonged fever (over 39.5°C) and severe respiratory symptoms | | |
| +++ The foals died after recurrent high fever or prolonged fever (over 40°C) and severe respiratory symptoms | | |

**TABLE 3 (Exp. 2)**

| Protection was elicited against natural infection after two immunizations with EHV-2 iscoms into foals | | | |
|---|---|---|---|
| Group | Number of animals | Number of immunisations | Number of animals () with symptoms and score of symptoms |
| I | 5 | non-vaccinated controls | (3) +++ |
| | | | (1) ++ |
| | | | (1) |
| II | 19 | 2* | (1) + |
| | | | (18)- |

| | | | |
|---|---|---|---|
| * The foals were vaccinated first at two weeks of age and then boosted at four weeks - No symptoms + Body temperature over 39.5-40.5°C, fever approximately one week, respiratory infection ++ Body temperature over 39.5, prolonged fever, serious respiratory passage symptoms +++ Dead after recurrent high fever or prolonged fever (over 40°C) with respiratory symptoms | | | |

**Table 4.**

| The serum antibody response of juvenile seals immunized with CDV iscom measured by virus neutralization seals in group II were from the coast outside Wales and encompass animals with and without detectable maternal antibodies to CDV | | |
|---|---|---|
| Name | CDV-VNT | CDV-VNT |
| Tigh | 20 | 20 |
| Sine | 20 | 20 |
| Matthew | 20 | 60 |
| Gittara | 20 | 20 |
| Bonnie | <20 | 20 |
| Rob | <20 | 20 |
| Pax | 20 | 60-180 |
| Fury | 20 | 20 |
| Benfro | <20 | 20 |
| Bolly | 20 | 20 |
| Sidney | <20 | 20 |
| Solomon | 20 | 60 |
| Jetty | <20 | 20 |

**Table 5.**

| The serum antibody response of juvenile seals immunized with CDV-iscoms measured by virusneutralisation. The seals were selected to have maternal antibodies to CDV | | |
|---|---|---|
| Name | First sample | Second sample |
| 95-09 | 30 | 30 |
| 95-10 | 100 | 300 |
| 95-15 | 100 | 300 |
| 95-11 | 30 | 30 |
| 95-58 | 30 | 10 |
| 95-27 | 30 | 30 |
| 96-12 | 20 | 60 |
| 95-30 | 30 | 30 |
| 95-39 | 100 | 100 |
| 95-47 | 10 | 30 |
| 95-04 | 100 | 100 |
| 95-50 | 100 | 100 |
| 95-69 | 30 | 10 |
| 95-59 | 300 | 1000 |
| 95-86 | 100 | 100 |
| 95-87 | 30 | 30 |
| 95-20 | 100 | 300 |
| 95-97 | 30 | 100 |
| 95-57 | 30 | 30 |
| 96-12 | 30 | 30 |
| 96-10 | 30 | 30 |
| 95-30 | 20 | 60 |
| 95-96 | 100 | 300 |

**Table 6**

| Immunization with Sendai virus iscoms induce immune response in neonate mice. | | | |
|---|---|---|---|
| Groups | Primary Immunization | Boostered | Ab response |
| 1 | 1 µg iscom/dose | 1 µg iscom/dose | ++ |
| 2 | 1 µg micelles/dose | 1 µg micelles/dose | - |
| 3a | No | 0.2 µg iscoms/dose | +/- |
| 3b | No | 0.2 µg micelles/dose | - |
| 4 | 0.2 µg iscoms/dose | 0.2 µg iscoms/dose | +++ |
| 5 | 0.2 µg micelles/dose | 0.2 µg micelles/dose | - |

**Table 7**

| Serum antibody response measured by HI of puppies vaccinated with killed parovirus adjuvanted with iscom-matrix (I) or a commercial vaccine (No symbol) at the age of 3 weeks (day 0) and a second time day 23. | | | | |
|---|---|---|---|---|
| Day of sampling after first vaccination HI-titres | | | | |
| Puppy Code No: | 0 | 10 | 23 | 33 |
| 9694 I | 40 | 20 | <10 | 2560 |
| 9695 | 20 | 10 | 10 | 160 |
| 9696 I | 20 | 20 | 160 | 5120 |
| 9697 | 10 | 10 | 10 | 160 |
| 9698 I | 20 | 10 | <10 | 2560 |
| 9699 | 40 | 20 | 10 | 40 |
| 9700 I | 10 | 20 | 20 | 5120 |

## Claims

1. Use of immunogenic complexes in the form of iscom comprising at least one glycoside, at least one lipid and at least one antigen or in the form of iscom-matrix comprising at least one glycoside and at least one lipid for preparing a vaccine that evokes immune response in juvenils individuals who have an immature immune system in that they have a reduced capacity to respond immunologically when they are exposed to antigenic stimuli or have a maternal immunity, i.e. antibodies from their mothers, or have both an immature immune system and a maternal immunity.

2. Use of an immunogenic complex according to claim 1, whereby the complex is composed of an iscom complex.

3. Use of an immunogenic complex according to claim 1, whereby the complex is composed of iscom-matrix and iscom-matrix are mixed with one or more antigens intended to elicit specific immune response to included antigen(s) or that iscom-matrix and antigens are prepared as separate entities (units) intended to be adminstered mixed or separately.

4. Use of an immunogenic complex according to any of claims 1-3, whereby the iscom or iscom-matrix antigen formulation is administered via mucus.

5. Use of an immunogenic complex according to claim 4, whereby the iscom or iscom-matrix antigen formulation is administered by oral, nasal, urogenital and/or rectal administration.

6. Use of an immunogenic complex according to any of claims 1-5, whereby the antigen(s) derive(s) from microorganisms.

7. Use of an immunogenic complex according to claim 6, whereby the antigen(s) derive(s) from viruses, bacteria or parasites.

8. Use of an immunogenic complex according to any of claims 1-7, whereby the antigen(s) is(are) produced by recombinant DNA technique or chemical synthesis.

9. Use of immunogenic complexes according to claims 6-8, whereby the antigen(s) is/are chosen from measles virus, canine distemper virus, parvovirus, herpes viruses, rotaviruses.

## Patentansprüche

1. Verwendung von immunogenen Komplexen als ISCOM, umfassend wenigstens ein Glykosid, wenigstens ein Lipid und wenigstens ein Antigen, oder als ISCOM-Matrix, umfassend wenigstens ein Glykosid und wenigstens ein Lipid, zur Herstellung eines Impfstoffs, der eine Immunreaktion bei jungen Einzellebewesen hervorruft, die ein unreifes Immunsystem besitzen, als sie eine verminderte Fähigkeit haben, immunologisch zu reagieren, wenn sie antigenen Stimuli ausgesetzt sind, oder die eine maternale Immunität haben, d.h. Antikörper ihrer Mutter, oder die sowohl ein unreifes Immunsystem als auch eine maternale Immunität haben.

2. Verwendung eines immunogenen Komplexes nach Anspruch 1, wobei sich der Komplex aus einem ISCOM-Komplex zusammensetzt.

3. Verwendung eines immunogenen Komplexes nach Anspruch 1, wobei sich der Komplex aus einer ISCOM-Matrix zusammensetzt und die ISCOM-Matrix mit ein oder mehreren Antigenen vermischt ist, die eine spezifische Immunreaktionen gegen beigefügte(s) Antigen(e) Antigene hervorrufen sollen, oder die ISCOM-Matrix und die Antigene als separate Elemente (Einheiten) zur Verabreichung in vermischter oder getrennter Form hergestellt sind.

4. Verwendung eines immunogenen Komplexes nach irgendeinem der Ansprüche 1-3, wobei die ISCOM- oder ISCOM-Matrix-Antigen-Formulierung via Mukus verabreicht wird.

5. Verwendung eines immunogenen Komplexes nach Anspruch 4, wobei die ISCOM- oder ISCOM-Matrix-Antigen-Formulierung durch orale, nasale, urogenitale und/oder rektale Verabreichung verabreicht wird.

6. Verwendung eines immunogenen Komplexes nach irgendeinem der Ansprüche 1-5, wobei das (die) Antigen(e) aus Mikroorganismen stammt (stammen).

7. Verwendung eines immunogenen Komplexes nach Anspruch 6, wobei das (die) Antigen(e) aus Viren, Bakterien oder Parasiten stammt (stammen).

8. Verwendung eines immunogenen Komplexes nach irgendeinem der Ansprüche 1-7, wobei das (die) Antigen(e) durch rekombinante DNA-Technologie oder chemische Synthese hergestellt ist (sind).

9. Verwendung immunogener Komplexe nach den Ansprüchen 6-8, wobei das (die) Antigen(e) aus Masernvirus, Hundestaupe-Virus, Parvovirus, Herpesviren und Rotaviren ausgewählt ist/sind.

## Revendications

1. Utilisation de complexes immunogènes sous forme d'iscom comprenant au moins un glycoside, au moins un lipide et au moins un antigène ou sous forme d'une matrice d'iscom comprenant au moins un glycoside et au moins un lipide pour préparer un vaccin qui déclenche une réponse immunitaire chez des individus juvéniles qui ont un système immunitaire immature en ce qu'ils ont une capacité réduite à réagir immunologiquement quand ils sont exposés à des stimuli antigéniques ou qui ont une immunité maternelle, c'est-à-dire des anticorps provenant de leur mère, ou qui ont à la fois un système immunitaire immature et une immunité maternelle.

2. Utilisation d'un complexe immunogène selon la revendication 1, où le complexe est composé d'un complexe d'iscom.

3. Utilisation d'un complexe immunogène selon la revendication 1, où le complexe est composé d'une matrice d'iscom et la matrice d'iscom est mélangée avec un ou plusieurs antigènes destinés à déclencher une réponse immunitaire spécifique à un ou des antigènes inclus ou la matrice d'iscom et les antigènes sont préparés sous forme d'entités (unités) séparées destinées à être administrées en mélange ou séparément.

4. Utilisation d'un complexe immunogène selon l'une quelconque des revendications 1 à 3 où la formulation d'iscom ou de matrice d'iscom et d'antigène est administrée par l'intermédiaire de mucus.

5. Utilisation d'un complexe immunogène selon la revendication 4, où la formulation d'iscom ou de matrice d'iscom et d'antigène est administrée par administration orale, nasale, urogénitale et/ou rectale.

6. Utilisation d'un complexe immunogène selon l'une quelconque des revendications 1 à 5, où le ou les antigènes proviennent de microorganismes.

7. Utilisation d'un complexe immunogène selon la revendication 6, où le ou les antigènes proviennent de virus, de bactéries ou de parasites.

8. Utilisation d'un complexe immunogène selon l'une quelconque des revendications 1 à 7, où le ou les antigènes est ou sont produits par génie génétique ou synthèse chimique.

9. Utilisation de complexes immunogènes selon les revendications 6 à 8, où le ou les antigènes est ou sont choisis parmi le virus de la rougeole, le virus de la maladie de Carré, le parvovirus, les herpèsvirus, les rotavirus.
